# EUROPEAN PATENT APPLICATION

(11) **EP 0 935 963 A2**
(43) Date of publication of application: **18.08.1999**
(21) Application number: 98308563.0
(22) Date of filing: 20.10.1998
(51) Int. Cl.: A61K 31/44, A61K 31/66, A61K 31/445, A61K 31/54, A61K 31/165, A61K 31/40, A61K 31/35

(54) **The use of MMP-13 selective inhibitors for the treatment of osteoarthritis and other matrix metalloproteinase-mediated disorders**

(30) Priority: 24.10.1997 US 62766 P
(71) Applicant: Pfizer Products Inc., Groton, CT 06340-5146 (US)
(72) Inventor: McClure, Kim Francis, Mystic, Connecticut 06355 (US); Lopresti-Morrow, Lori Lynn, Salem, Connecticut 06420 (US); Mitchell, Peter Geoffrey, Mystic, Connecticut 06355 (US); Reeves, Lisa Marie, Stonington, Connecticut 06378 (US); Reiter, Lawrence Alan, Mystic, Connecticut 06355 (US); Robinson, Ralph Pelton, Gales Ferry, Connecticut 06355 (US); Yocum, Sue Ann, Baltic, Connecticut 06330 (US)
(74) Representative: McMunn, Watson Palmer

(57) **Abstract**

The present invention relates to the use of matrix metalloproteinase-13 selective agents for the treatment or prevention of osteoarthritis and other matrix metalloproteinase-mediated disorders.

## Description

### BACKGROUND OF THE INVENTION

The present invention relates to the use of matrix metalloproteinase inhibitors which display specificity for matrix metalloproteinase-13 versus matrix metalloproteinase-1. These compounds are useful in the treatment or prevention of osteoarthritis, rheumatoid arthritis and cancer.

Osteoarthritis and rheumatoid arthritis are joint diseases which are characterized by cartilage damage and loss of joint function, which may eventually culminate in joint replacement. One of the principal biochemical events in such joint diseases is damage to and subsequent loss of type II collagen, the predominant articular cartilage collagen. Type II collagen is extremely resistant to proteolytic cleavage, with the collagenases of the matrix metalloproteinase (MMP) family being the only mammalian enzymes with the ability to cleave the native, helical type II, collagen present in normal cartilage.

Three type II collagen degrading matrix metalloproteinases are currently known, MMP-1, MMP-8 and MMP-13. These matrix metalloproteinases are also known as collagenases 1, 2 and 3, respectively. Matrix metalloproteinase-1, collagenase-1, is expressed throughout the body by a wide variety of connective tissues including osteoarthritic cartilage and synovium. Mitchell et al., J. Clin. Invest., 97, 761-768 (1996) and Wolfe et al., Arthritis Rheum., 36, 1540-1547 (1993). Matrix metalloproteinase-8, collagenase-2, was initially thought to be expressed exclusively by neutrophils. However, recent evidence demonstrates that low levels of MMP-8 mRNA and protein are present in human cartilage and it has been suggested that this enzyme may participate in aggrecan degradation, Chubinskaya et al., Lab. Invest., 74, 232-240 (1993) and Cole et al., J. Biol. Chem., 271, 11023-11026 (1996). Matrix metalloproteinase-13, collagenase-3, originally reported in a breast carcinoma cell line, is the most recent member of this class to be discovered, Freije et al., J. Biol. Chem., 269, 16766-16773 (1994) . The present inventors independently cloned collagenase-3 from human osteoarthritic cartilage and have demonstrated that this new matrix metalloproteinase is expressed at substantial levels in human osteoarthritic cartilage, Mitchell et al., J. Clin. Invest., 97, 761-768 (1996).

Matrix metalloproteinase inhibitors, i.e. compounds which broadly inhibit MMPs including MMP-1 MMP-8 and MMP-13, are known and have been used to demonstrate that non-selective MMP inhibitors block cartilage collagen degradation in *in vitro* cartilage explant systems. Nixon et al., Int. J. Tiss. React., 13, 237-243 (1991); Mitchell et al., Annals. New York Acad. Sci., 732, 395-397 (1994), and Mort et al., Matrix., 13, 95-102 (1993).

Until recently, MMP-1 was the only collagenase known to be expressed by cartilage and other connective tissues and, as a result, has been the subject of the most collagenase research. As stated above, comprehensive tissue distribution studies have demonstrated that MMP-1 can be expressed by a variety of tissues especially those involved in connective tissue collagen homeostasis such as skin, gingiva, meniscus, tendon and ligament.

The present inventors have now discovered that MMP-13, contrary to MMP-1, is not expressed in any of these tissues, (e.g., skin, gingiva, meniscus, tendon and ligament), even under conditions (e.g., interleukin-1 stimulation) in which MMP-1 is substantially upregulated. The only tissue with significant MMP-13 expression is cartilage.

Furthermore, the present inventors have also demonstrated that, *in vitro,* collagenase-3 has ten fold greater activity against type II collagen than does collagenase-1, J. Clin. Invest., supra. However, *in vitro* activity of the collagenases is not necessarily indicative of the biological role of each of these agents, because both enzymes exist in a pro-active form that is converted to the active species by mechanisms that have yet to be clearly elucidated *in vivo.* In addition, natural inhibitors (e.g., the tissue inhibitors of metalloproteinases or TIMPs) can differentially regulate MMP activity. Thus, in the absence of data demonstrating that compounds with selectivity for MMP-13 versus MMP-1 eliminate type II collagen degradation, it would be speculative to conclude that such a selective inhibitor could be useful to treat or prevent osteoarthritis and other matrix metalloproteinase mediated disorders.

The present inventors have surprisingly discovered that even though collagenase-1 and collagenase-3 are present in activated cartilage, inhibitors with specificity for collagenase-3 versus collagenase-1 blocked substantially all type II collagen degradation at concentrations indicative of collagenase-3 inhibition but not collagenase-1 inhibition. This is so surprising, because one of ordinary skill in the art would have expected that a MMP-13 selective inhibitor would either have no effect or would have only a partial effect on collagen degradation. This is so, because MMP-1 was thought to be the principal collagen degrading enzyme in cartilage and thus the uninhibited MMP-1 would have been expected to continue degrading collagen. However, as the data below demonstrate, such is not the case.

Furthermore, recent clinical trial data for marimastat, a non-selective MMP-1 and MMP-13 inhibitor, revealed significant dose-related connective tissue side effects, likely due to inhibition of normal connective tissue collagen tumover by systemic inhibition of MMP-1, Proceedings of ASCO, 15, 490 (1996). Such connective tissue toxicity significantly limits the therapeutic applications of such MMP-1/MMP-13 inhibitors. Based on the tissue distribution studies, that demonstrate that MMP-13 is almost exclusively expressed in cartilage while MMP-1 is broadly expressed, it is expected that systemic inhibition of MMP-13 will not produce the connective tissue toxicity limiting the use of non-selective MMP-1/MMP-13 inhibitors. Thus, in conjunction with the collagenase inhibition data referred to above, the present inventors have discovered that compounds with MMP-13 versus MMP-1 activity are surprisingly superior inhibitors of cartilage collagen degradation that will act without producing systemic connective tissue toxicity.

Matrix metalloproteinase inhibitors, i.e. agents which broadly inhibit matrix metalloproteinases 1, 8 and 13, have been described in the literature. Specifically, PCT publications WO 96/33172, published October 24, 1996, and WO 96/27583, published March 7, 1996, refer to hydroxamic acids which inhibit matrix metalloproteinases.

PCT Application No. PCT/US95/07166, filed June 07, 1995 (which corresponds to United States Patent Application No. 60/017850, filed May 20, 1996), refers to other hydroxamic acids which inhibit matrix metalloproteinases.

United States Patent Application No. 60/021,652, filed July 12, 1996; United States Patent Application No. 60/024,675, filed August 23, 1996; United States Patent Application No. 60/021,959, filed July 18, 1996; United States Patent Application No. 60/037,600, filed February 11, 1997; United States Patent Application No. 60/037,402, filed February 7, 1997; United States Patent Application No. 60/036,857, filed February 3, 1997; and United States Patent Application No. 60/034,535, filed January 6, 1997, refer to other hydroxamic acids, glutaramides or phosphinates which also inhibit matrix metalloproteinases.

European Patent Application 606,046, published July 13, 1994, refers to aryl sulfonamido-substituted hydroxamic acids and states that they are a useful as matrix metalloproteinase inhibitors.

PCT publication WO 90/05719, published May 31, 1990, refers to other hydroxamic acids and states that they are useful as MMP inhibitors.

European Patent Publication 780,386, published June 25, 1997, refers to novel matrix metalloproteinase inhibitors. The authors state that, based on tissue distribution of the collagenases, collagenase-3 is the major participant in the degradation of the cartilage collagen matrix and, therefore, inhibitors selective for collagenase-3 over collagenase-1 would be preferred for the treatment of diseases associated with cartilage erosion, such as arthritis. However, the tissue distribution data in this application was very limited and did not include many of the connective tissues that are known producers of MMP-1. In addition, no evidence was presented that inhibitors with selectivity for MMP-13 versus MMP-1 inhibited cartilage collagen degradation.

German Patent Publication 19,501,032, published July 18, 1996, presents claims to the treatment of rheumatic disorders by inactivating collagenase-3. The authors provide no data to indicate that collagenase-3 was in fact inhibited.

### SUMMARY OF THE INVENTION

The present invention relates to a method of treating or preventing a disorder or condition that can be treated or prevented by selectively inhibiting collagenase-3 with respect to collagenase-1 (preferably without producing systemic connective tissue toxicity) in a mammal, preferably a human, comprising administering to said mammal requiring such treatment or prevention a collagenase-3 inhibiting effective amount of a collagenase-3 selective inhibitor, or pharmaceutically acceptable salts thereof.

Thus a first aspect of the invention is the use of a collagenase-3 selective inhibitor or a pharmaceutically acceptable salt in the preparation of a medicament for the treatment or prophylaxis of a disorder or condition which can be treated or prevented by selectively inhibiting collagenase-3.

Preferably the condition of disorder is osteoarthritis.

The present invention also relates to a method of treating or preventing osteoarthritis in a mammal, preferably a human, comprising administering to said mammal requiring such treatment or prevention a collagenase-3 inhibiting effective amount of a compound selected from the group consisting of:
1-([4-(4-fluorophenoxy)benzenesulfonyl]-pyridin-3-ylmethyl-amino)-cyclopentanecarboxylic acid,
2-([4-(4-fluorophenoxy)benzensulfonyl]-pyridin-3-ylmethyl-amino)-N-hydroxy-2 methyl-propionamide,
(4-benzyl-benzyl)-[2-(2,2-dimethyl-1-methylcarbamoylproplycarbamoyl)-6-phenoxy-hexyl]-phosphinic acid],
2-amino-3-[4-(4-fluorophenoxy)benzenesulfonyl]-N-hydroxypropionamide,
N-hydroxy-2-[(4-benzyloxy-benzensulfonyl)-pyridin-3-ylmethyl-amino]-acetamide,
(4-benzyl-benzyl)-(2-[2-(4-methoxy-phenyl)-1-methylcarbamoylethylcarbamoyl]-6-phenoxy-hexyl)-phosphinic acid
3-[4-(4-fluorophenoxy)benzenesulfonyl]-2, N-dihydroxypropionamide,
2-(1-[4-(4-fluorophenoxy)benzenesulfonyl]-cyclobutyl)-2, N-dihydroxy-acetamide,
3-(4-phenoxybenzenesulfonyl)-7-oxa-bicyclo[2.2.1]heptane-2-carboxylic acid hydroxyamide,
2-[4-(4-fluorophenoxy)benzenesulfonyl-amino]-N-hydroxy-2-methyl-propionamide,
1-[4-(4-fluorobenzyloxy)-benzenesulfonyl]-2-hydroxycarbamoylpiperidine-4-carboxylic acid,
4-(4'-chlorobiphenyl-4-yl)-2-[2-(1,3-dioxo-1,3-dihydro-isoindol-2-yl]-4-oxobutyric acid,
4-[4-(4-chlorophenoxy)benzensulfonylmethyl]-tetrahydropyran-4-carboxylic acid hydroxyamide,
(S)-α-[2-(4'-chloro[1,1'-biphenyl]-4-yl )-2-oxoethyl]-1,3-dihydro-1, 3-dioxo-2*H*-isoindole-2-butanoic acid;
4-[4-(4-chlorophenoxy)phenylsulfonylmethyl]-tetrahydropyran-4-(N-hydroxycarboxamide), and
3(S)-N-hydroxy-4-(4-((pyrid-4-yl)oxy)benzenesulfonyl)-2,2-dimethyl-tetrahydro-2H-1,4-thiazine-3-carboxamide,
or pharmaceutically acceptable salts thereof.

### DETAILED DESCRIPTION OF THE INVENTION

Collagenase inhibitors, wherein the MMP-13/MMP-1 selectivity ratio is not known, can be prepared according to methods well known to those of ordinary skill in the art. Specifically, collagenase inhibitors with broad activity and their methods of preparation have been described in WO 96/33172 and WO 96/27583; PCT Application No. PCT/US95/07166 filed June 07,1995 (which corresponds to United States Patent Application No. 60/017,850); PCT Application No. PCT/B95/00427, filed June 6 1995; EP-A-818442 (United States Patent Application No. 60/021,652); WO 98/07697 (United States Patent Applicaton No. 60/024,675); WO 98/03516 (United States Patent Application No. 60/021,959); WO 98/34918 (United States Patent Application No 60/037,600); WO 98/34915 (United States Patent Application No. 60/037,402); WO 98/33768. (United States Patent Applicaton No. 60/036,857). WO 98/30566. (United States Patent Application No. 60/034,535); EP-A-606,046; WO 90/05719; and EP-A-780,386. All of the foregoing references are incorporated herein by reference in their entirety.

Collagenase-3 (matrix metalloproteinase-13) selective inhibitors as used herein refer to agents which exhibit at least a 100 fold selectivity for the inhibition of collagenase-3 enzyme activity over collagenase-1 enzyme activity and a potency of less than 100 nM as defined by the IC₅₀ results from the MMP-13/MMP-1 fluoroescence assays described below. Collagenase-3 selective inhibitors can be identified by screening collagenase inhibitors, prepared according to the patents and publications described above, through the MMP-13/MMP-1 fluorescence assays described below and selecting those agents with MMP-13/MM)-1 inhibition IC₅₀ ratios of 100 or greater and potency of less than 100 nM. MMP-13 (collagenase-3) selective inhibitors may possess differential activity towards other MMP's, aggrecanase, TACE, ADAM-10, ADAM-12,.
ADAMTS-1 or collagenase 2. One of ordinary skill in the art would know how to assay for these various metalloenzymes.

Collagenase inhibitors as used herein refer to compounds with collagenase inhibiting activity wherein the MMP-13/MMP-1 selectivity is not known.

Non-selective collagenase inhibitors as used herein refer to agents which exhibit less than a 100 fold selectivity for the inhibition of collagenase-3 enzyme activity over collagenase-1 enzyme activity or a potency of more than 100nM as defined by the IC₅₀ results from the MMP-13/MMP-1 fluorescence assays described below.

The ability of collagenase inhibitors to inhibit collagenase activity is well known in the an The present inventors are the first to discover the ability of collagenase-3 (matrix metalloproteinase-13) selective inhibitors, preferably the compounds listed above as preferred compounds of the invention or their pharmaceutically acceptable salts (hereinafter also referred to as the active compounds) to inhibit type II cartilage collagen degradation. The following experiments demonstrate: (1) that the compounds do indeed have selectivity for MMP-13 versus MMP-1; (2) MMP-13 selective compounds inhibit substantially all of the collagenase activity released from IL-1 stimulated cartilage explants; and (3) MMP-13 selective compounds inhibit substantially all type II cartilage collagen degradation in cartilage explants.

### EXPERIMENT 1

### Inhibition of Human Collagenase (MMP-1)

Human recombinant collagenase-1 is activated with trypsin. The amount of trypsin is optimized for each lot of collagenase-1 but a typical reaction uses the following ratio: 5 µg trypsin per 100 µg of collagenase.. The trypsin and collagenase are incubated at room temperature for 10 minutes then a five fold excess (50 mg/10 mg trypsin) of soybean trypsin inhibitor is added.

Stock solutions (10 mM) of inhibitors are made up in dimethylsulfoxide and then diluted using the following Scheme:
10 mM → 120 µM → 12 µM → 1.2 µM → 0.12 µM

Twenty-five microliters of each concentration is then added to triplicate wells of a 96 well microfluor plate. The final concentration of inhibitor will be a 1:4 dilution after addition of enzyme and substrate. Positive controls (enzyme, no inhibitor) and negative controls (no enzyme no inhibitors) are also run in triplicate on each plate.

Collagenase-1 is diluted to 240 ng/ml and 25 µl is then added to appropriate wells of the microfluor plate. Final concentration of collagenase in the assay is 60 ng/ml.

Substrate (DNP-Pro-Cha-Gly-Cys(Me)-His-Ala-Lys(NMA)-NH₂) is made as a 5 mM stock in dimethyl sulfoxide and then diluted to 20 µM in assay buffer. The assay is initiated by the addition of 50 µl substrate per well of the microfluor plate to give a final concentration of 10 µM.

Fluorescence readings (360 nM excitation, 460 nM emission) are taken at time 0 and then at 20 minute intervals. The assay is conducted at room temperature with a typical assay time of 3 hours.

Fluorescence versus time is then plotted for both the blank and collagenase containing samples (data from triplicate determinations is averaged). A time point that provides a good signal (at least 5 fold over the blank) and that is on a linear part of the curve (usually around 120 minutes) is chosen to determine IC₅₀ values. The zero time is used as a blank for each compound at each concentration and these values are subtracted from the 120 minute data. Data is plotted as inhibitor concentration versus % control (inhibitor fluorescence divided by fluorescence of collagenase alone x 100). IC₅₀'s are determined from the concentration of inhibitor that gives a signal that is 50% of the control.

If IC₅₀'s are reported to be less than 0.03 µM, then the inhibitors are assayed at concentrations of 0.3 µM, 0.03 µM, and 0.003 µM.

### Inhibition of MMP-13

Human recombinant MMP-13 is activated with 2 mM APMA (p-aminophenyl mercuric acetate) for 1.5 hours, at 37°C and is then diluted to 240 mg/ml in assay buffer (50 mM Tris, pH 7.5, 200 mM sodium chloride, 5mM calcium chloride, 20µM zinc chloride, 0.02% Brij 35). Twenty-five microliters of diluted enzyme is added per well of a 96 well microfluor plate. The enzyme is then diluted in a 1:4 ratio in the assay by the addition of inhibitor and substrate to give a final concentration in the assay of 60 ng/ml.

Stock solutions (10 mM) of inhibitors are made up in dimethylsulfoxide and then diluted in assay buffer as per the inhibitor dilution scheme for inhibition of human collagenase-1. Twenty-five microliters of each concentration is added to triplicate wells of a microfluor plate. The final concentrations in the assay are 30 µM, 3 µM, 0.3 µM, and 0.03 µM.

Substrate (Dnp-Pro-Cha-Gly-Cys(Me)-His-Ala-Lys(NMA)-NH₂) is prepared as for inhibition of human collagenase (MMP-1) and 50 ml is added to each well to give a final assay concentration of 10 µM. Fluorescence readings (360 nM excitation; 450 nM emission) are taken at time 0 and every 5 minutes for 1 hour.

Positive controls and negative controls are set up in triplicate as outlined in the MMP-1 assay.

IC₅₀'s are determined as per inhibition of human collagenase (MMP-1). If IC₅₀'s are reported to be less than 0.03 mM, inhibitors are then assayed at final concentrations of 0.3 µM, 0.03 µM, 0.003 µM and 0.0003 µM.

Matrix metalloproteinase-13 selective inhibtors are agents that demonstrate an IC₅₀ of less than 100 nM in the MMP-13 assay described above. Furthermore, the IC₅₀ ratio of MMP-13/MMP-1 inhibition must be greater than 100 fold.

Application of the above referenced protocols to the broad spectrum inhibitor, Example 1 (prepared according to Example 2 from United States Patent No. 4,599,361, issued July 8, 1986), and two MMP-13 selective compounds, Examples 2 and 3 (prepared according to Example 2 from World Patent Application Serial No. PCT/IB95/00427, filed June 6, 1995, and Example 11 from United States Patent Application Serial No. 09/892,417, filed July 14, 1997 claiming priority from United States Patent Application No. 60/021,959, filed July 18, 1996, respectively) yielded the data reported in Table 1.

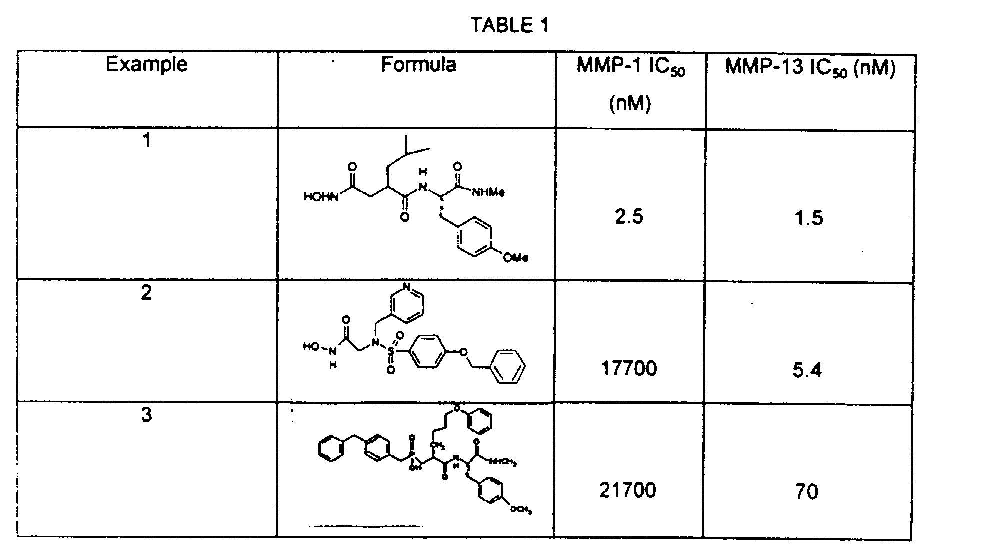

### EXPERIMENT 2

### Collagen film MMP-13 Assay

The purpose of this second experiment is to: (1) demonstrate that the active agents inhibit collagen degradation and (2) to determine which active collagenases are present in the conditioned medium from cartilage explants undergoing resorption. Only collagenases can degrade helical collagen whereas peptides, such as the substrate described in Experiment 1 above, can potentially be degraded by a variety of proteases. The method described below can be used to verify the fluorescence assay data with respect to the selectivity of inhibitors for MMP-13 versus MMP-1. The principal advantage of this assay over the fluorescence assay is that the natural substrate is used (i.e. collagen) instead of an artificial substrate (e.g., fluorescent peptide substrates). In addition, since collagen is the substrate and since only collagenases can degrade this substrate, collagenase activity in complex mixtures of enzymes (e.g., the conditioned medium from cartilage explants) can be determined. One of ordinary skill in the art will understand that this assay, described below, can be adjusted in many ways to suit the particular needs of an experimenter.

Rat type I collagen is radiolabeled with ¹⁴C acetic anhydride (T.E. Cawston and A.J. Barrett, Anal. Biochem., 99, 340-345 (1979)) and used to prepare 96 well plates containing radiolabeled collagen films (Barbara Johnson-Wint, Anal. Biochem., 104, 175-181 (1980)). When a solution containing collagenase is added to the well, the enzyme cleaves the insoluble collagen which unwinds and is thus solubilized. Collagenase activity is directly proportional to the amount of collagen solubilized, determined by the proportion of radioactivity released into the supernatant as measured in a standard scintillation counter. Collagenase inhibitors are, therefore, compounds which reduce the radioactive counts released with respect to the controls with no inhibitor present. One specific embodiment of this assay is described in detail below.

For determining the selectivity of compounds for MMP-13 versus MMP-1 using collagen as a substrate, the following procedure is used. Recombinant human proMMP-13 or proMMP-1 is activated according to the procedures outlined in Experiment 1. The activated MMP-13 or MMP-1 is diluted to 0.6 ug/ml with buffer ( 50 mM Tris pH 7.5, 150 mM NaCI, 10 mM CaCl₂, 1 uM ZnCl₂, 0.05% Brij-35, 0.02% sodium azide).

Stock solutions of test compound (10mM) in dimethylsulfoxide are prepared. Dilutions of the test compounds in the Tris buffer, above, are made to 0.2, 2.0, 20, 200, 2000 and 20000 nM.

100 µl of appropriate drug dilution and 100 µl of diluted enzyme are pipetted into wells of a 96 well plate containing collagen films labeled with ¹⁴C-collagen. The final enzyme concentration is 0.3 µg/ml while the final drug concentration is 0.1, 1.0, 10, 100, 1000 nM. Each drug concentration and control is analyzed in triplicate. Triplicate controls are also run for the conditions in which no enzyme is present and for enzyme in the absence of any compound.

The plates are incubated at 37°C for a time period such that around 30 - 50% of the available collagen is solubilized - determined by counting additional control wells at various time points. In most cases around 9 hours of incubation are required. When the assay has progressed sufficiently, the supernatant from each well is removed and counted in a scintillation counter. The background counts (determined by the counts in the wells with no enzyme) are subtracted from each sample and the % release calculated in relation to the wells with enzyme only and no inhibitor. The triplicate values for each point are averaged and the data graphed as percent release versus drug concentration. IC₅₀'s are determined from the point at which 50% inhibition of release of radiolabeled collagen is obtained. IC₅₀'s in this assay (type I collagen) for Examples 1-3 are shown in Table 2.

To determine the identity of the active collagenases in cartilage conditioned medium, assays were carried out using collagen as a substrate, cartilage conditioned medium containing collagenase activity and inhibtors of varying selectivity. The cartilage conditioned medium was collected during the time at which collagen degradation was occurring and thus is representative of the collagenases responsible for the collagen breakdown. Assays were carried out as outlined above except that instead of using recombinant MMP-13 or recombinant MMP-1, cartilage conditioned medium was the enzyme source. The same inhibitors were assayed and their IC₅₀'s are also listed in Table 2. As expected, the equipotent MMP-1/MMP-13 inhibitor, Example 1, was a potent inhibitor of the cartilage collagenase conditioned medium activity. Since the compound is an equipotent inhibitor of both collagenases, it is not possible to determine if one or both enzymes are present as an active species in the conditioned medium. However, surprisingly, the two structurally divergent MMP-13 selective compounds Examples 2 and 3, were also potent inhibitors of the cartilage conditioned medium collagenase activity with IC₅₀'s indicative of MMP-13 being the predominantly active collagenase and not MMP-1.

**TABLE 2**

| Example | MMP-1 Fluor. IC₅₀ (nM) | MMP-1 Type I Collagen IC₅₀ (nM) | MMP-13 Fluor. IC₅₀ (nM) | MMP-13 Type 1 Collagen IC₅₀ (nM) | Cartilage Conditioned Medium IC₅₀ (nM) |
|---|---|---|---|---|---|
| 1 | 2.5 | 3.5 | 1.5 | 1.5 | 10 |
| 2 | 17700 | 22000 | 5.4 | 52 | 220 |
| 3 | 21700 | 40000 | 70 | 40 | 400 |

### EXAMPLE 3

### IL-1 Induced Cartilage Collagen Degradation From Bovine Nasal Cartilage

This third experiment demonstrates that agents with MMP-13/MMP-1 selectivity inhibit substantially all cartilage collagen degradation in cartilage explants. This assay uses bovine nasal cartilage explants which are commonly used to test the efficacy of various compounds to inhibit either IL-1 induced proteoglycan degradation or IL-1 induced collagen degradation. Bovine nasal cartilage is a tissue that is very similar to articular cartilage, i.e. chondrocytes surrounded by a matrix that is primarily type II collagen and aggrecan. The tissue is used because it: (1) is very similar to articular cartilage, (2) is readily available, (3) is relatively homogeneous, and (4) degrades with predictable kinetics after IL-1 stimulation. In addition, the inventors have found that in bovine nasal cartilage, as in human articular cartilage, IL-1 stimulates upregulation of both MMP-1 and MMP-13 mRNA and protein.

Two variations of this assay have been used to assay compounds. Both variations give similar data. The two variations are described below:

### Variation 1

Three plugs of bovine nasal cartilage (approximately 2 mm diameter x 1.5 mm long) are placed into each well of a 24 well tissue culture plate. One ml of serumless medium is then added to each well. Compounds are prepared as 10 mM stock solutions in DMSO and then diluted appropriately in serumless medium to final concentrations, e.g., 50, 500 and 5000 nM. Each concentration is assayed in triplicate.

Human recombinant IL-1α (5ng/mL) (IL-1) is added to triplicate control wells and to each well containing drug. Triplicate control wells are also set up in which neither drug nor IL-1 are added. The medium is removed and fresh medium containing IL-1 and the appropriate drug concentrations is added on days 6, 12, 18 and 24 or every 3 - 4 days if necessary. The media removed at each time point is stored at -20°C for later analysis. When the cartilage in the IL-1 alone wells has almost completely resorbed (about day 21), the experiment is terminated. The medium, is removed and stored. Aliquots (100 ul) from each well at each time point are pooled, digested with papain and then analyzed for hydroxyproline content. Background hydroxyproline (average of wells with no IL-1 and no drug) is subtracted from each data point and the average calculated for each triplicate. The data is then expressed as a percent of the IL-1 alone average value and plotted. The IC₅₀ is determined from this plot.

### Variation 2

The experimental set-up is the same as outlined above in Variation 1, until day 12. On day 12, the conditioned medium from each well is removed and frozen. Then one ml of phosphate buffered saline (PBS) containing 0.5 µg/ml trypsin is added to each well and incubation continued for a further 48 hours at 37°C. After 48 hours incubation in trypsin, the PBS solution is removed. Aliquots (50 µl) of the PBS/trypsin solution and the previous two time points (days 6 and 12) are pooled. hydrolyzed and hydroxyproline content determined. Background hydroxyproline (average of wells with no IL-1 and no drug) is subtracted from each data point and the average calculated for each triplicate. The data is then expressed as a percent of the IL-1 alone average value and plotted. The IC₅₀ is determined from this plot. In this variation, the time course of the experiment is shortened considerably. The addition of trypsin for 48 hours after 12 days of IL-1 stimulation likely releases any type II collagen that has been damaged by collagenase activity but not yet released from the cartilage matrix. In the absence of IL-1 stimulation, trypsin treatment produces only low background levels of collagen degradation in the cartilage explants.

The data for Examples 1-3 in the cartilage explant assay are summarized in Table 3. For comparison, their IC₅₀'s versus type I collagen are also included. It can be seen from Table 3 that inhibition of type II cartilage collagen degradation correlates very well with inhibition of MMP-13 but not at all with inhibition of MMP-1. Thus, not only does the inhibitory profile of the active collagenase in the conditioned medium correlate with MMP-13 inhibition, inhibition of IL-1 induced type II cartilage collagen degradation also correlates with MMP-13 inhibition.

**TABLE 3**

| Example | MMP-1 Type I Collagen IC₅₀ (nM) | MMP-13 Type I Collagen IC₅₀ (nM) | Cartilage Conditioned medium IC₅₀ (nM) | Cartilage Explant Assay IC₅₀(nM) | Cartilage Explant Assay IC₅₀(nM) |
|---|---|---|---|---|---|
| | | | | Variation 1 | Variation 2 |
| 1 | 3.5 | 1.5 | 10 | 60* | 50 |
| 2 | 22000 | 52 | 220 | 900** | 550 |
| 3 | 40000 | 40 | 400 | | 830 |

| | | | | | |
|---|---|---|---|---|---|
| * Average IC50 (variation 1 and 2) Example 1 = 90 ± 35 nM | | | | | |
| ** Average IC₅₀ (variation 1 and 2) Example 2 = 725 ± 400 nM. | | | | | |

For administration to mammals, including humans, for the inhibition of matrix metalloproteinases, a variety of conventional routes may be used including oral, parenteral (e.g., intravenous, intramuscular or subcutaneous), buccal, anal and topical. In general, the active compound will be administered at dosages between about 0.01 and 25 mg/kg body weight of the subject to be treated per day, preferably from about 0.3 to 5 mg/kg. Preferably the active compound will be administered orally or parenterally. However, some variation in dosage will necessarily occur depending on the condition of the subject being treated. The person responsible for administration will, in any event, determine the appropriate dose for the individual subject.

The active compounds can be administered in a wide variety of different dosage forms, in general, the therapeutically effective compounds of this invention are present in such dosage forms at concentration levels ranging from about 5.0% to about 70% by weight

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelation and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof. In the case of animals, they are advantageously contained in an animal feed or drinking water in a concentration of 5-5000 ppm, preferably 25 to 500 ppm.

For parenteral administration (intramuscular, intraperitoneal, subcutaneous and intravenous use) a sterile injectable solution of the active ingredient is usually prepared. Solutions of a therapeutic compound of the present invention in either sesame or peanut oil or in aqueous propylene glycol may be employed. The aqueous solutions should be suitably adjusted and buffered, preferably at a pH of greater than 8, if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readity accomplished by standard pharmaceutical techniques well known to those skilled in the art In the case of animals, compounds can be administered intramuscularly or subcutaneously at dosage levels of about 0.1 to 50 mg/kg/day, advantageously 0.2 to 10 mg/kg/day given in a single dose or up to 3 divided doses.

The active compounds may also be formulated in rectal compositions such as suppositories or retention enemas, e.g., containing conventional suppository bases such as cocoa butter or other glycerides.

For intranasal administration or administration by inhalation, the active compounds are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

## Claims

1. Use of a collagenase-3 selective inhibitor or a pharmaceuticaly acceptable salt in the prepararation of a medicament for the treatment or prophylaxis of a disorder or condition which can be treated or prevented by selectively inhibiting collagenase-3.

2. Use as claimed in claim 1 wherein the medicament can treat said condition or disorder without producing systemic connective tissue toxicity.

3. Use as claimed in claims 1 or 2 wherein the condition or disorder is osteoarthritis.

4. Use as claimed in any one of claims 1 to 4 wherein the collagenase-3 inhibitor is selected from the group consisting of;
1-([4-(4-fluorophenoxy)benzenesulfonyl]-pyridin-3-ylmethylamino)-cyclopentanecarboxylic acid,
2-([4-(4-fluorophenoxy)benzenesulfonyl]-pyridin-3-ylmethylamino]-N-hydroxy-2 methyl-propionamide,
(4-benzyl-benzyl)-[2-(2,2-dimethyl-1-methylcarbamoylpropylcarbamoyl)-6-phenoxy-hexyl]-phosphinic acid,
2-amino-3-[4-(4-fluorophenoxy)benzenesulfonyl-N-hydroxypropionamide,
N-hydroxy-2-[(4-phenoxy-benzenesulfonyl)-pyridin-3-ylmethylamino]-acetamide,
(4-benzyl-benzyl)-(2-[2-(4-methoxy-phenyl)-1-methylcarbamoylethylcarbamoyl]-6-phenoxy-hexyl]-phosphinic acid,
3-[4-(4-fluorophenoxy)benzenesulfonyl]-2, N-dihydroxypropionamide,
2-[1-[4-(4-fluorophenoxy)benzenesulfonyl]-cyclobutyl]-2, N-dihydroxy-acetamide,
3-(4-phenoxybenzenesulfonyl)-7-oxa-bicyclo[2.2.1]heptane-2-carboxylic acid hydroxyamide,
2-[4-(4-fluorophenoxy)benzenesulfonyl-amino]-N-hydroxy-2-methyl-propionamide,
1-[4-(4-fluorobenzyloxy)-benzenesulfonyl]-2-hydroxycarbamoylpiperidine-4-carboxylic acid
4-(4'-chlorobiphenyl-4-yl)-2-[2-(1,3-dioxo-1,3-dihydro-isoindol-2-yl)ethyl]-4-oxobutyric acid,
4-[4-(4-chlorophenoxy)benzenesulfonylmethyl]-tetrahydropran-4-carboxylic acid hydroxyamide,
(S)-α[2-(4'-chloro[1,1'-biphenyl]-4-yl)-2-oxoethyl]-1,3-dihydro-1, 3-dioxo-2H-isoindole-2-butanoic acid;
4-[4-(4-chlorophenoxy)phenylsulfonylmethyl]-tetrahydropyran-4-(N-hydroxycarboxamide), and
3(S)-N-hydroxy-4-(4-((pyrid-4-yl)oxy)benzenesulfonyl)-2,2-dimethyl-tetrahydro-2H-1,4-thiazine-3-carboxamide,
or a pharmaceutically acceptable salt thereof.
